# EUROPEAN PATENT APPLICATION

(11) **EP 3 618 073 A1**
(43) Date of publication of application: **04.03.2020**
(21) Application number: 18192076.0
(22) Date of filing: 31.08.2018
(51) Int. Cl.: G16H 10/60, G16H 40/20, H04L 9/00

(54) **A METHOD AND APPARATUS FOR HYBRID TRUST MANAGEMENT FOR HEALTH RECORDS AUDIT**

(71) Applicant: Koninklijke Philips N.V., 5656 AE Eindhoven (NL)
(72) Inventor: QU, Jin, 5656 AE Eindhoven (NL); GE, Xin, 5656 AE Eindhoven (NL); XIA, Fubiao, 5656 AE Eindhoven (NL)
(74) Representative: de Haan, Poul Erik

(57) **Abstract**

Various embodiments relate to a method for health record audit verification, the method including the steps of creating an audit trail and sending the audit trail to a plurality of roles, computing, by each of the plurality of roles, a plurality of weighted credible numbers by multiplying a weighted credit by a total number of individuals for each of the plurality of roles, determining, by each of the plurality of roles, whether the sum of the plurality of weighted credible numbers is greater than a predetermined threshold and updating, by each of the plurality of roles, an audit trail chain on a Merkle tree.

## Description

### FIELD OF THE INVENTION

This disclosure relates generally to a method for verifying data, and more specifically, but not exclusively, to a method for health records audit verification.

### BACKGROUND OF THE INVENTION

Distributed Management Task Force ("DMTF") Cloud Auditing Data Federation ("CADF") provides an audit trail collecting architecture.

Blockchain provides a method for a decentralized system based on peer-to-peer network and anonymous roles.

### SUMMARY OF THE INVENTION

A brief summary of various embodiments is presented below. Embodiments address a method and apparatus for hybrid trust management for health records audit.

A brief summary of various example embodiments is presented. Some simplifications and omissions may be made in the following summary, which is intended to highlight and introduce some aspects of the various example embodiments, but not to limit the scope of the invention.

Detailed descriptions of example embodiments adequate to allow those of ordinary skill in the art to make and use the inventive concepts will follow in later sections.

Various embodiments relate to a method for health record audit verification, the method including the steps of creating an audit trail and sending the audit trail to a plurality of roles, computing, by each of the plurality of roles, a plurality of weighted credible numbers by multiplying a weighted credit by a total number of individuals for each of the plurality of roles, determining, by each of the plurality of roles, whether the sum of the plurality of weighted credible numbers is greater than a predetermined threshold and updating, by each of the plurality of roles, an audit trail chain on a Merkle tree.

In an embodiment of the present disclosure, the plurality of roles are a medical facility, a medical authority, an insurance company, an auditor and a patient.

In an embodiment of the present disclosure, the time for verifying the audit trail decreases when the weighted credit increases and the time for verifying the audit trail increases when the weighted credit decreases.

In an embodiment of the present disclosure, the auditor and the patient generate a public key and a private key.

In an embodiment of the present disclosure, the medical facility, the medical authority and the insurance company have a private key generated and signed by a certificate authority.

In an embodiment of the present disclosure, the verified audit trails are stored in a Merkle root and indexed by a patient identifier.

In an embodiment of the present disclosure, for the medical facility, a computational capability of the medical facility is limited by an upper threshold, N_{UVMP} of a total number of staff verifying the audit trail, N_{MP} being less than the predetermined threshold.

In an embodiment of the present disclosure, for the medical facility, a weighted credible number, N_{CMP} is less than the predetermined threshold such that when N_{MP} is less than or equal to N_{UVMP}, then N_{CMP} is equal to N_{MP} and when N_{MP} is greater than or equal to N_{UVMP}, then N_{CMP} is equal to N_{UVMP}.

In an embodiment of the present disclosure, for the insurance company, the weighted credit for the insurance company, C_{WI} is equal to a weighted credible number, Nci.

In an embodiment of the present disclosure, for the medical authority, the weighted credit for the medical authority, C_{WA} is equal to a weighted credible number, N_{CA}.

In an embodiment of the present disclosure, for the auditor, a computational capability of the auditor is limited by an upper threshold, N_{UVAD} of a total number of auditors verifying the audit trail, N_{MAD} being less than the predetermined threshold.

In an embodiment of the present disclosure, for the auditor, a weighted credible number, N_{CAD} is less than the predetermined threshold such that when N_{AD} is less than or equal to N_{UVAD}, then N_{CAD} is equal to N_{AD} and when N_{AD} is greater than or equal to N_{UVAD}, then N_{CAD} is equal to N_{UVAD}.

In an embodiment of the present disclosure, for the patient, the weighted credit for the patient, C_{WP} is equal to a weighted credible number, N_{CP}.

In an embodiment of the present disclosure, when N_{CMP} + N_{CI} + N_{CA} + N_{UVMP} is greater than or equal to the predetermined threshold, the verification is valid.

In an embodiment of the present disclosure, when N_{CMP} + C_{WI} + N_{CAD} + C_{WP} is greater than or equal to the predetermined threshold, the verification is valid.

### BRIEF DESCRIPTION OF THE DRAWINGS

The accompanying figures, where like reference numerals refer to identical or functionally similar elements throughout the separate views, together with the detailed description below, are incorporated in and form part of the specification, and serve to further illustrate example embodiments of concepts found in the claims and explain various principles and advantages of those embodiments.

These and other more detailed and specific features are more fully disclosed in the following specification, reference being had to the accompanying drawings, in which:
FIG. 1 illustrates a block diagram of a hybrid trust management system architecture of the current embodiment;
FIG. 2 illustrates a data structure of an audit trail of the current embodiment;
FIG. 3 illustrates a block diagram of a method for adding an audit record to an audit trial chain of the current embodiment;
FIG. 4 illustrates the relationship of the Merkle root and each audit trails of the current embodiment;
FIG. 5 illustrates a data structure of a signature of a verified audit trail of the current embodiment; and
FIG. 6 illustrates a block diagram of a real-time data processing system of the current embodiment.

### DETAILED DESCRIPTION OF EMBODIMENTS

It should be understood that the figures are merely schematic and are not drawn to scale. It should also be understood that the same reference numerals are used throughout the figures to indicate the same or similar parts.

The descriptions and drawings illustrate the principles of various example embodiments. It will thus be appreciated that those skilled in the art will be able to devise various arrangements that, although not explicitly described or shown herein, embody the principles of the invention and are included within its scope. Furthermore, all examples recited herein are principally intended expressly to be for pedagogical purposes to aid the reader in understanding the principles of the invention and the concepts contributed by the inventor to furthering the art and are to be construed as being without limitation to such specifically recited examples and conditions. Additionally, the term, "or," as used herein, refers to a non-exclusive or (i.e., and/or), unless otherwise indicated (e.g., "or else" or "or in the alternative"). Also, the various embodiments described herein are not necessarily mutually exclusive, as some embodiments can be combined with one or more other embodiments to form new embodiments. Descriptors such as "first," "second," "third," etc., are not meant to limit the order of elements discussed, are used to distinguish one element from the next, and are generally interchangeable.

When there is medical dispute, medical facilities need to provide evidence to support their claims. These medical facilities have their own health IT systems and use audit trails to record actions on health records. How to make the public trust their evidence and enhance the transparency of their operation is a problem.

Maintenance of medical records has become necessary in the health care field. For example, when there is medical dispute, medical facilities, such as a hospital, may be required to provide evidence to support their claims to insurance companies or other parties. These medical facilities often have their own health information technology ("IT") systems and use audit trails to record actions on health records. However, public trust in these health records from these medical facilities is often low and enhancements to the transparency of the medical facilities operations of medical recording is required.

Therefore, to enhance the transparency of medical facilities and increase the public's trust in the medical recording of these medical facilities, these embodiments provide a method for an audit trail chain based on an audit trail management system, which allows the public to verify the audit trail using computing efforts.

Specifically, these embodiments provide a decentralized audit trail verification system. In these embodiments, all of the audit trails produced by a medical facility are stored in an audit trail chain, which can be verified by different departments of this medical facility, medical authorities, insurance companies, professional auditors, patients registered at this medical facility, and other stakeholders in the records.

The medical facility, the medical authorities or insurance companies may have their digital certificates, but patients, auditors, or employees of the medical facility may or may not have their own digital certificates. All of these roles may take part in the audit trail verification process, which builds transparency and trust of the medical recording of the medical facility.

The embodiments describe herein provide a dynamic M-conformation method based on peers' credits.

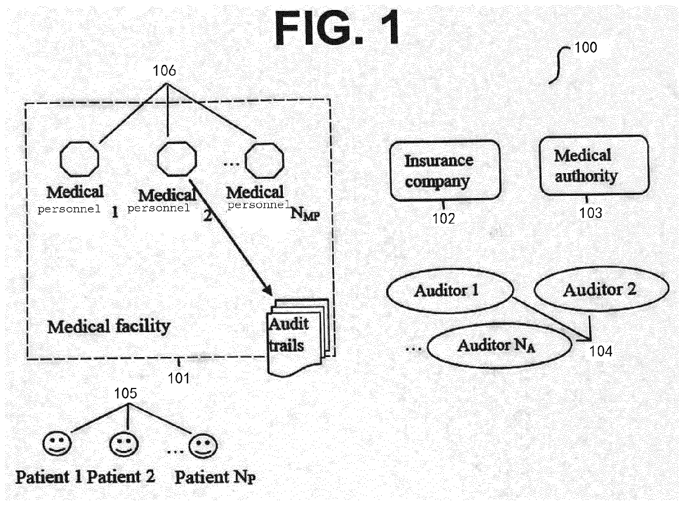

FIG. 1 illustrates a block diagram of a hybrid trust management system architecture 100 of the current embodiment.

All roles involved in the audit trail generation and verification process are illustrated in FIG. 1.

The hybrid trust management system 100 includes a medical facility 101, an insurance company 102, a medical authority 103, auditors 104 and patients 105. Medical personnel 106 may be involved in generating and verifying audit trails.

The insurance company 102 provides payment to the medical facility 101 for patients 105. However, when medical disputes or fraud occur, the income of the insurance company 101 is affected. Medical disputes and fraud damage the benefits of other patients 105 because the insurance company 101 may increase the insurance fee to mitigate the money loss caused by medical disputes and fraud. Therefore, the insurance company 101 may be motivated to verify the audit trails and decrease health records mistakes in order to prevent medical disputes or fraud.

Similarly, as a patient 105 seeks to protect themselves from any medical mistake, the patient 105 may need to ensure all audit trails about their medical records are correct and accurate. Patients 105 registered at the medical facility 101 also may be motivated to prevent medical fraud which may decrease their medical cost, on average, therefore they have the motivation to verify audit trails.

Similarly, a medical authority has a responsibility to minimize medical mistakes and medical fraud, improve the quality of medical services, and verify audit trails. Further, professional auditors 104 may also join the process of verifying the audit trails.

FIG. 1 is a hybrid trust management system architecture 101 which is not a peer-to-peer network because the credibility of an insurance company, a medical authority, professional auditors, a medical facility and its medical personal and patients are different. The cost incurred by damaging their credibility is different for each entity, and their computing capability is different. Furthermore, the responsibility to maintain a trusted ecosystem is different for each entity. If the credit of a role is high, the required number of times of verification is low.

In the current embodiment, a threshold M is used. If the total creditable verification for an audit trail is greater than threshold M, then the audit trail is valid.

The number of individuals for each entity participating in the verification computation is a value, N. Each N value is weighted by multiplying the N value by a weighted credit, C. The product of this calculation is known as the weighted credible number.

A medical facility 101 generates an audit trail and has an interest in verifying the audit trail is correct by engaging the staff of the medical facility 101 to participate in the verification computation.

The total number of staff participating in a verification computation is defined as N_{MP}.

The upper threshold of a total number of staff participating in a verification computation is N_{UVMP}, where N_{UVMP} < M, which is used to limit the computation capability of a medical facility 101.

The weighted credible number is N_{CMP}, where N_{CMP} < M. If, N_{MP} ≤ N_{UVMP}, N_{CMP} = N_{MP}; else N_{CMP} = N_{UVMP}. The weighted credible number, for example, is calculated by the total number of staff participating in a verification computation, N_{MP} multiplied by a weighted credit, C.

An insurance company 102 may also join the audit trail verification process. The valid number of insurance companies which are participating in the verification computation may be 1. If the weighted credit for insurance company 101 is C_{WI}, assuming there is only one insurance company participating in the verification computation then, the weighted creditable number of verification for the insurance company is Nci which is equal C_{WI}.

A medical authority 103 may also join the audit trail verification process. There may be one or more authorities involved in the audit trail verification, however, if there is only one medical authority 103, the valid number of medical authorities which are participating in the verification computation is 1. If the weighted credit for the medical authority 103 is C_{WA}, assuming there is only one medical authority participating in the verification computation then, the weighted creditable number of verification for the medical authority is N_{CA} which is equal to C_{WA}.

For auditors 104, audit trail verification may increase their credit and may join the verification computation. The total number of auditors 104 joining a verification computation is denoted by N_{MAD}. The upper threshold of the valid number is N_{UVAD}, where N_{UVAD} < M, which is used to limit the computation capability of a medical facility 101. The weighted credible number is N_{CAD}, where, N_{CAD} < M. If, N_{AD} ≤ N_{UVAD}, N_{CAD} = N_{AD}; else N_{CAD} = N_{UVAD}.

For patients 105, if the audit trail involves a patient 105, the patient 105 may have a level of background knowledge and may join the verification computation, however, there may be other patients 105 who may not have enough knowledge or may not be permitted to join the verification computation process because of privacy concerns. Therefore, the valid number of patients who are participating in the verification computation is 1. If the weighted credit for the patient 105 is C_{WP}, assuming there is only one patient participating in the verification computation then, the weighted creditable number of verification is N_{CP} which is equal to C_{WP}.

The relationship between these weighted creditable numbers of verification is shown by the following inequality:

N_{CMP} + N_{CI} + N_{CA} + N_{UVMP} ≥ *M,* or N_{CMP} + C_{WI} + N_{CAD} + C_{WP} ≥ *M,* which means that if the sum of all the creditable numbers is greater than or equal to *M,* the verification is valid. The second inequality assumes that the number of auditors, insurance companies and medical authorities is equal to 1.

When the sum of all of the weighted credible numbers, which indicates each of the entities which participated in the verification computation, is greater than or equal to a threshold value, then the verification computation is valid. If the sum of all of the weighted credible numbers is less than a threshold value, then the verification computation is not valid.

When the verification computation is valid, the audit trail may be added to the Merkle tree.

The medical facility 101, the medical authority 103 and the insurance company 102 may acquire certificates from a public Certificate Authority. The medical facility 101 may encourage medical personnel from the medical facility 101 to join the audit trail verification process to show operational transparency and trust.

Anonymity of the medical personnel of the medical facility 101, the medical authority 103 or the insurance company 102 in the verification process may not be kept.

Therefore, the medical facility 101, the medical authority 103 and the insurance company 102 may sign the audit trail with their private key granted by a public Certificate Authority, after they verify the audit trail.

However, for auditors 104 and patients 105, it may be necessary to maintain their anonymity in the verification process, therefore auditors 104 and patients 105 may solve a proof of work computation to ensure that the verification process remains secure.

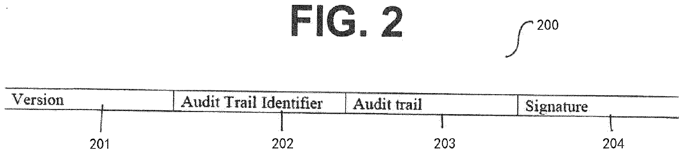

Therefore, when there are changes to a patient's medical records, one of the medical personnel will generate one audit trail as illustrated in FIG. 2.

FIG. 2 illustrates a data structure 200 of an audit trail 203 of the current embodiment.

The version 201 is used to denote the protocol version, the audit trial identifier 202 is used to link this audit trail to an audit trail chain, and the signature 204 is generated by one of the medical personnel of the medical facility.

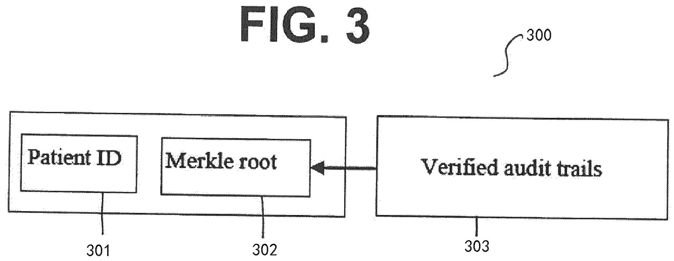

If an audit trail 203 is valid, the audit trail 203 is added to an audit trail chain as illustrated in FIG. 3.

FIG. 3 illustrates a block diagram of a method for adding an audit record to an audit trial chain 300 of the current embodiment.

The Patient identification ("ID") 301 is used to index a patient. A Merkle root 302 is used to store all audit trails. The verified audit trails 303 stores all verified audit trails.

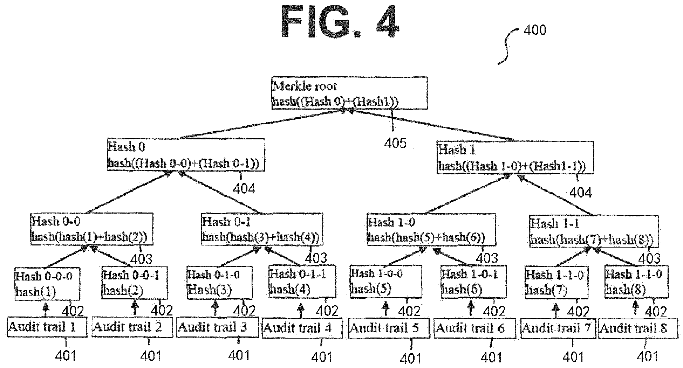

FIG. 4 illustrates the relationship 400 of the Merkle root and each audit trail of the current embodiment. By using a Merkle root, a user may calculate a hashed result as a branch node from the leaf node, however, the reverse calculation cannot be performed. Therefore, once an audit trail has been verified and added to the Merkle tree, this audit trail cannot be changed.

The relationship 400 may be used to prevent any verified audit trial from being tampered.

In FIG. 4, audit trails 1-8 401 are identified.

Hash(x) 402, where x = 1, ..., 8, are a hash value of each audit trail x 401. For example, hash(1) 402 is a hash value for audit trail 1 401.

Hash(hash(x)+hash(x+1)) 403, where x = 1, 3, 5, and 7, is a hash value of the previous hash(x) values 402. For example, hash(hash(1)+hash(2)) 403 is a hash value of the previous hash(1) 402 and hash (2) 402.

Hash((Hash 0-0)+(Hash 0-1)) and Hash((Hash 0-1)+(Hash 1-1)) 404 are hash values of the previous hash values 403. For example, hash((Hash 0-0)+Hash 0-1)) is a hash value of the previous Hash 0-0 403 and Hash 0-1 403.

Hash((Hash 0)+(Hash 1)) 405 is known as the Hash root or Merkle root and is a hash value of the previous hash values 404. For example, Hash(Hash 0)+(Hash 1) 405 is a hash value for the previous Hash 0 404 and Hash 1 404.

The audit trail identifier is a hashed value of the patient ID and the Merkle root as illustrated in FIG. 2.

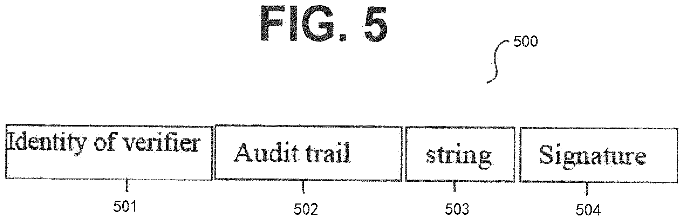

For example, if a node verifies the audit trail (as illustrated in FIG. 2), the audit trail is claimed by that person, and that person signs the claim as illustrated in FIG. 5.

FIG. 5 illustrates a data structure 500 of a signature of a verified audit trail of the current embodiment.

The data structure 500 includes the identity of the verifier 501 which is the name or some of ID of the person who verified the audit trail, the audit trail 502, the string 503 from the Merkle root, and the signature 504, which is the signature of the verifier (i.e., the Certificate Authority certificate).

Medical personnel, the insurance company, and the medical authority who own their Certificate Authority certificate may sign the verified audit trail with their private keys.

Auditors and patients, who do not disclose their identities may sign the verified audit trail by solving a proof of work computation.

For the nodes which verify an audit trail anonymously, to prevent any of these nodes from generating false audit trail chain, a method of Blockchain may be used by providing an audit trail with a proof-of-work. Any user who solves the proof-of-work computation is considered to be trusted and may anonymously verify the audit trail. The proof-of-work is used to decrease the number of false audit trail by increasing the computing cost in the proof-of-work. If the user cannot solve the proof-of-work computation.

After receiving a verified audit trail, a node may compute if the total number of received verified audit trail is greater than or equal to *M.* If number is greater than or equal to *M,* the node will update the local stored audit trail chain.

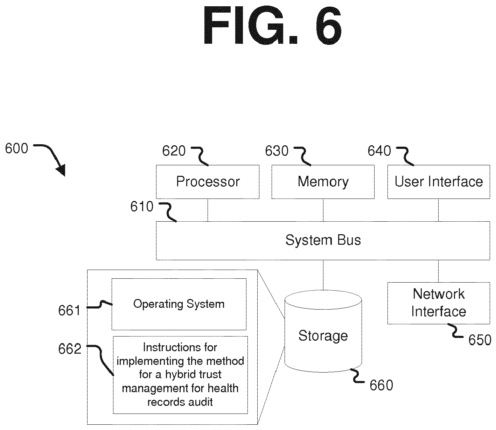

FIG. 6 illustrates an exemplary hardware diagram 600 for implementing a method for hybrid trust management for health records audit. As shown, the device 600 includes a processor 620, memory 630, user interface 640, network interface 650, and storage 660 interconnected via one or more system buses 610. It will be understood that FIG. 1 constitutes, in some respects, an abstraction and that the actual organization of the components of the device 600 may be more complex than illustrated.

The processor 620 may be any hardware device capable of executing instructions stored in memory 630 or storage 660 or otherwise processing data. As such, the processor may include a microprocessor, field programmable gate array (FPGA), application-specific integrated circuit (ASIC), or other similar devices.

The memory 630 may include various memories such as, for example L1, L2, or L3 cache or system memory. As such, the memory 630 may include static random access memory (SRAM), dynamic RAM (DRAM), flash memory, read only memory (ROM), or other similar memory devices.

The user interface 640 may include one or more devices for enabling communication with a user such as an administrator. For example, the user interface 340 may include a display, a mouse, and a keyboard for receiving user commands. In some embodiments, the user interface 640 may include a command line interface or graphical user interface that may be presented to a remote terminal via the network interface 650.

The network interface 650 may include one or more devices for enabling communication with other hardware devices. For example, the network interface 650 may include a network interface card (NIC) configured to communicate according to the Ethernet protocol. Additionally, the network interface 650 may implement a TCP/IP stack for communication according to the TCP/IP protocols. Various alternative or additional hardware or configurations for the network interface 650 will be apparent.

The storage 660 may include one or more machine-readable storage media such as read-only memory (ROM), random-access memory (RAM), magnetic disk storage media, optical storage media, flash-memory devices, or similar storage media. In various embodiments, the storage 660 may store instructions for execution by the processor 620 or data upon with the processor 620 may operate. For example, the storage 660 may store a base operating system 661 for controlling various basic operations of the hardware 600 and instructions for implementing the method for hybrid trust management for health records audit 662.

It will be apparent that various information described as stored in the storage 660 may be additionally or alternatively stored in the memory 630. In this respect, the memory 630 may also be considered to constitute a "storage device" and the storage 660 may be considered a "memory." Various other arrangements will be apparent. Further, the memory 630 and storage 660 may both be considered "non-transitory machine-readable media." As used herein, the term "non-transitory" will be understood to exclude transitory signals but to include all forms of storage, including both volatile and non-volatile memories.

While the host device 600 is shown as including one of each described component, the various components may be duplicated in various embodiments. For example, the processor 620 may include multiple microprocessors that are configured to independently execute the methods described herein or are configured to perform steps or subroutines of the methods described herein such that the multiple processors cooperate to achieve the functionality described herein. Further, where the device 600 is implemented in a cloud computing system, the various hardware components may belong to separate physical systems. For example, the processor 620 may include a first processor in a first server and a second processor in a second server.

It should be apparent from the foregoing description that various exemplary embodiments of the invention may be implemented in hardware. Furthermore, various exemplary embodiments may be implemented as instructions stored on a non-transitory machine-readable storage medium, such as a volatile or non-volatile memory, which may be read and executed by at least one processor to perform the operations described in detail herein. A non-transitory machine-readable storage medium may include any mechanism for storing information in a form readable by a machine, such as a personal or laptop computer, a server, or other computing device. Thus, a non-transitory machine-readable storage medium may include read-only memory (ROM), random-access memory (RAM), magnetic disk storage media, optical storage media, flash-memory devices, and similar storage media and excludes transitory signals.

It should be appreciated by those skilled in the art that any blocks and block diagrams herein represent conceptual views of illustrative circuitry embodying the principles of the invention. Implementation of particular blocks can vary while they can be implemented in the hardware or software domain without limiting the scope of the invention. Similarly, it will be appreciated that any flow charts, flow diagrams, state transition diagrams, pseudo code, and the like represent various processes which may be substantially represented in machine readable media and so executed by a computer or processor, whether or not such computer or processor is explicitly shown.

Accordingly, it is to be understood that the above description is intended to be illustrative and not restrictive. Many embodiments and applications other than the examples provided would be apparent upon reading the above description. The scope should be determined, not with reference to the above description or Abstract below, but should instead be determined with reference to the appended claims, along with the full scope of equivalents to which such claims are entitled. It is anticipated and intended that future developments will occur in the technologies discussed herein, and that the disclosed systems and methods will be incorporated into such future embodiments. In sum, it should be understood that the application is capable of modification and variation.

The benefits, advantages, solutions to problems, and any element(s) that may cause any benefit, advantage, or solution to occur or become more pronounced are not to be construed as a critical, required, or essential features or elements of any or all the claims. The invention is defined solely by the appended claims including any amendments made during the pendency of this application and all equivalents of those claims as issued.

All terms used in the claims are intended to be given their broadest reasonable constructions and their ordinary meanings as understood by those knowledgeable in the technologies described herein unless an explicit indication to the contrary in made herein. In particular, use of the singular articles such as "a," "the," "said," etc. should be read to recite one or more of the indicated elements unless a claim recites an explicit limitation to the contrary.

The Abstract of the Disclosure is provided to allow the reader to quickly ascertain the nature of the technical disclosure. It is submitted with the understanding that it will not be used to interpret or limit the scope or meaning of the claims. In addition, in the foregoing Detailed Description, it can be seen that various features are grouped together in various embodiments for the purpose of streamlining the disclosure. This method of disclosure is not to be interpreted as reflecting an intention that the claimed embodiments require more features than are expressly recited in each claim. Rather, as the following claims reflect, inventive subject matter lies in less than all features of a single disclosed embodiment. Thus the following claims are hereby incorporated into the Detailed Description, with each claim standing on its own as a separately claimed subject matter.

## Claims

1. A method for health record audit verification, the method comprising the steps of:
creating an audit trail and sending the audit trail to a plurality of roles;
computing, by each of the plurality of roles, a plurality of weighted credible numbers by multiplying a weighted credit by a total number of individuals for each of the plurality of roles;
determining, by each of the plurality of roles, whether the sum of the plurality of weighted credible numbers is greater than a predetermined threshold; and
updating, by each of the plurality of roles, an audit trail chain on a Merkle tree..

2. The method for health record audit verification of claim 1, wherein the plurality of roles are a medical facility, a medical authority, an insurance company, an auditor and a patient.

3. The method for health record audit verification of claim 1, wherein the time for verifying the audit trail decreases when the weighted credit increases and the time for verifying the audit trail increases when the weighted credit decreases.

4. The method for health record audit verification of claim 2, wherein the auditor and the patient generate a public key and a private key.

5. The method for health record audit verification of claim 2, wherein the medical facility, the medical authority and the insurance company have a private key generated and signed by a certificate authority.

6. The method for health record audit verification of claim 1, wherein the verified audit trails are stored in a Merkle root and indexed by a patient identifier.

7. The method for health record audit verification of claim 2, wherein for the medical facility, a computational capability of the medical facility is limited by an upper threshold, N_{UVMP} of a total number of staff verifying the audit trail, N_{MP} being less than the predetermined threshold.

8. The method for health record audit verification of claim 7, wherein for the medical facility, a weighted credible number, N_{CMP} is less than the predetermined threshold such that when N_{MP} is less than or equal to N_{UVMP}, then N_{CMP} is equal to N_{MP} and when N_{MP} is greater than or equal to N_{UVMP}, then N_{CMP} is equal to N_{UVMP}.

9. The method for health record audit verification of claim 8, wherein for the insurance company, the weighted credit for the insurance company, C_{WI} is equal to a weighted credible number, N_{CI}.

10. The method for health record audit verification of claim 9, wherein for the medical authority, the weighted credit for the medical authority, C_{WA} is equal to a weighted credible number, N_{CA}.

11. The method for health record audit verification of claim 10, wherein for the auditor, a computational capability of the auditor is limited by an upper threshold, N_{UVAD} of a total number of auditors verifying the audit trail, N_{MAD} being less than the predetermined threshold.

12. The method for health record audit verification of claim 11, wherein for the auditor, a weighted credible number, N_{CAD} is less than the predetermined threshold such that when N_{AD} is less than or equal to N_{UVAD}, then N_{CAD} is equal to N_{AD} and when N_{AD} is greater than or equal to N_{UVAD}, then N_{CAD} is equal to N_{UVAD}.

13. The method for health record audit verification of claim 12, wherein for the patient, the weighted credit for the patient, C_{WP} is equal to a weighted credible number, N_{CP}.

14. The method for health record audit verification of claim 13, wherein when N_{CMP} + N_{CI} + N_{CA} + N_{UVMP} is greater than or equal to the predetermined threshold, the verification is valid.

15. The method for health record audit verification of claim 13, wherein when N_{CMP} + C_{WI} + N_{CAD} + C_{WP} is greater than or equal to the predetermined threshold, the verification is valid.
